(19) Europäisches Patentamt
European Patent Office
Office européen des brevets

(11) Publication number: **0 006 741**
**B1**

(12) **EUROPEAN PATENT SPECIFICATION**

(45) Date of publication of patent specification: **12.01.83**

(51) Int. Cl.³: **A 61 K 7/08,** A 61 K 7/50, A 61 K 7/15

(21) Application number: **79301206.3**

(22) Date of filing: **21.06.79**

(54) **Self-emulsifying cosmetic base.**

(30) Priority: **23.06.78 US 918260**

(43) Date of publication of application:
**09.01.80 Bulletin 80/1**

(45) Publication of the grant of the patent:
**12.01.83 Bulletin 83/2**

(84) Designated Contracting States:
**DE FR GB IT**

(56) References cited:
**BE - A - 516 807**
**FR - A - 2 125 531**

**SOAP/COSMETICS/CHEMICAL SPECIALITIES, August 1975, vol. 51, no. 8, New York USA "Waterless Hand Cleaner" page 108.**

(73) Proprietor: **HENKEL CORPORATION**
**4620 West 77th Street**
**Minneapolis Minnesota 55435 (US)**

(72) Inventor: **Chun, Ho-Ming**
**1721-19th Avenue N.W.**
**New Brighton, Minnesota 55112 (US)**
Inventor: **Melby, Allan Lee**
**3912 Enchanted Drive, N.W.**
**Andover, Minnesota 55303 (US)**

(74) Representative: **Watkins, Arnold Jack et al,**
**European Patent Attorney Frank B. Dehn & Co.**
**Imperial House 15-19 Kingsway**
**London WC2B 6UZ (GB)**

**EP 0 006 741 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Self-emulsifying cosmetic base

The present invention is concerned with cosmetic bases for use in the manufacture of cosmetic compositions in the form of oil-in-water emulsions.

Nitrogen containing amphoteric surfactants such as those utilised in the present invention are described in U.S. Patent 2,816,920 issued to Anderson on December 17, 1957. Disclosures of related amphoteric surfactants include U.S. Patent 3,430,641 issued March 4, 1969 to Newman. Further disclosures of such materials are found in U.S. Patent 2,929,788 issued March 22, 1960 to Freese et al.

Related compounds for use in cosmetic compositions such as shaving soaps and shampoos are disclosed in U.S. Patent 2,468,012 issued to Isbell on April 10, 1949. Similar related compounds are disclosed in U.S. Patent 2,814,643 issued to Aelony on November 26, 1957.

A later patent to Freese, i.e. U.S. Patent 3,093,591 issued June 11, 1963, discloses the use of mixtures of amphoteric surfactants as are employed in the present invention. An interesting disclosure of two-phase liquid detergent compositions is found in U.S. Patent 3,533,955 issued October 13, 1970 to Pader et al.

Various cosmetic compositions containing nitrogen containing amphoteric surfactants and/or fatty alcohols are disclosed in COSMEDIA® Product Sheets FC—5, HB—4, HB—5, MB—1 and SC—1 available from the Cosmedia group of Henkel Corporation (formerly General Mills Chemicals, Inc.) of Minneapolis, Minnesota, U.S.A.; such compositions include shaving creams, cleansing creams, hand creams, hand and body lotions and soap-free pigmented emulsions. The foregoing Product Sheets do not however disclose the production of cosmetic bases which are self-emulsifying in the sense that this term is hereinafter defined.

French patent application publication No. 2125531 (L'Oreal) discloses foamable cosmetic compositions such as bases for shampoo and bubble baths. These compositions comprise a surfactant and one or more of the monohydric alcohols decan-1-ol, dodecan-1-ol and tetradecan-1-ol. Compositions containing dodecanol and sodium laurimino dipropionate in ratios of 1/18:1 and 3/10:1 are disclosed. The monohydric alcohols in the L'Oreal compositions serve to improve the foaming characteristics of the compositions. There is however no disclosure of the production of solid form cosmetic bases which are self-emulsifying in the sense that this term is hereinafter defined.

The present invention is based upon the findings that certain nitrogen-containing surfactants and fatty alcohols when combined in certain proportions not hitherto used provide novel cosmetic bases which are self-emulsifying (as hereinafter defined) and which can be used to produce emulsions at lower energy cost than that which would be involved if the same nitrogen-containing surfactants and fatty alcohols were separately incorporated into an emulsion composition.

According to the present invention, there is thus provided a solid form cosmetic base having self-emulsifying properties and comprising:

a) a mixture of compounds of the formula:

$$MOOCCH_2CH_2NRCH_2CH_2COOM$$

wherein R is a $C_{10}$ to $C_{24}$ fatty alkyl group and M consists of salt forming cations and hydrogen in a respective weight ratio of from 1:1 to $1:10^{-10}$; and

b) a saturated $C_{10}$ to $C_{24}$ fatty alcohol; the weight ratio of component (a) to component (b) being from 1/10:1 to 2/13:1.

Throughout the specification and claims, percentages and ratios are by weight unless otherwise indicated. Temperatures are indicated by degrees Celsius unless otherwise stated.

The self-emulsifying cosmetic bases according to the invention have been found to require relatively low energy input during their preparation and also to have relatively low energy requirements for the formation of cosmetic compositions in emulsion form upon the addition of the cosmetic base to oil and water.

As previously stated, the present invention deals with a self-emulsifying cosmetic base. The term "self-emulsifying" is used herein to mean that the cosmetic base has the property of forming an emulsion upon addition of the cosmetic base to oil and water; this is despite the fact that neither component (a) nor component (b) alone has this property.

It has been found that the use of a self-emulsifying cosmetic base according to the invention with oil and water to form an emulsion is more efficient and involves lower energy costs than if the two components (a) and (b) of the self-emulsifying cosmetic base are separately added to the oil and water.

It has furthermore been found that emulsions formed at the lower temperatures which can be used with the self-emulsifying cosmetic bases according to the invention are considerably more stable than emulsions formed at the higher temperatures needed where the components (a) and (b) are separately added to oil and water. By way of theory it is believed that once an emulsion is obtained any further energy input can cause the emulsion to become unstable. That is, one may not routinely heat a mixture which is in the process of becoming an emulsion to any particular higher temperature followed by lowering the temperature of the

emulsion and expect the same properties to be present in that emulsion as are present in a similar emulsion not heated to the higher temperature.

It has thus been found that the cosmetic base according to the invention enables components (a) and (b), neither of which will self-emulsify a water and oil system alone, to be presented in a solid form which is self-emulsifiable and can conveniently be used by the cosmetic manufacturer.

The energy requirements for preparing an oil-in-water emulsion from the solid form cosmetic base comprising only the nitrogen containing amphoteric surfactant and the fatty alcohol are considerably lower than by forming an emulsion through random addition of the various ingredients. Moreover, emulsions formed from the composition of the present invention tend to be more stable than similar emulsions formed in other ways.

The first component to be discussed in forming the solid form cosmetic base is the nitrogen containing amphoteric surfactant. The nitrogen containing amphoteric surfactant is a compound of the formula:

$$MOOCCH_2CH_2NRCH_2CH_2COOM$$

wherein M consists of salt forming cations and hydrogen. The weight ratio of the salt forming cation to the hydrogen should be from 1:1 to $1:10^{-10}$, preferably $1:10^{-1}$ to $1:10^{-9}$, most preferably from $1:10^{-2}$ to $1:10^{-8}$. R is a $C_{10}$ to $C_{24}$ fatty alkyl radical as later described.

In general the nitrogen containing amphoteric surfactant contains the salt forming cation and hydrogen in a particular ratio such that one mole of the nitrogen containing amphoteric surfactant dissolved in a liter of water will exhibit a pH between 2.5 and 9.5.

It has been found that a ratio of the salt forming cation to the hydrogen value of M in accordance with the invention provides a beneficial effect in emulsifying water and oil for the cosmetic product. That is, at least a portion of the self-emulsifying property of the solid form cosmetic base is due to the particular balance of the salt forming cation and hydrogen in the nitrogen containing amphoteric surfactant.

Preferably the salt forming cation is selected from sodium, potassium, ammonium and alkyl $(C_{1-3})$ derivatives of ammonium either fully or partially replacing the hydrogen atoms on the ammonium. Most preferably the salt forming cation is sodium.

The alkyl group R in the nitrogen containing amphoteric surfactant of component (a) preferably contains from 12 to 20 and is most preferably from 12 to 18 carbon atoms in length. R is preferably naturally derived such as from beef stock or coconut oil thereby having a large amount of lauryl, myristyl, cetyl and stearyl radicals present.

The second component in the solid form cosmetic base is a saturated $C_{10}$ to $C_{24}$ fatty alcohol. Preferably the fatty alcohol contains from 12 to 18 carbon atoms. It is also highly desirable that the saturated fatty alcohol be monohydric to ensure a high degree of stability when forming an emulsion. More preferably the natural alcohols are commensurate with the naturally derived groups of the nitrogen containing amphoteric surfactant, i.e. each having the same number of carbon atoms.

The solid form cosmetic base contains the nitrogen containing amphoteric surfactant and the saturated fatty alcohol in a respective weight ratio of from 1/10:1 to 2/13:1, preferably from 1/9:1 to 1/7:1.

It has also been found that, by combining components (a) and (b) to form the self-emulsifying cosmetic base according to the invention, considerably less amounts of the nitrogen containing amphoteric surfactant of component (a) are required than is the case if components (a) and (b) are separately added, thereby lowering the cost of that component in the finished cosmetic product with no loss in the effectiveness of the emulsifying properties.

Additional components which may optionally be included with the self emulsifying cosmetic base but which are not essential include all manner of cosmetic ingredients such as perfumes, water, emollients, surfactants and humectants.

The components of the present invention are conveniently combined to form the self-emulsifying cosmetic base by warming the fatty alcohol component to 75°C until it becomes clear and completely melted and then separately heating the nitrogen containing amphoteric surfactant of component (a) to 70°C. It should be recognised immediately that the only reason for heating the normally liquid nitrogen containing amphoteric surfactant is to ensure that it is thoroughly dispersed in the fatty alcohol.

It has been found best to add the nitrogen containing amphoteric surfactant to the fatty alcohol with continuous agitation i.e., mild shear conditions until the mixture turns completely clear. Thereafter, the mixture of the nitrogen containing amphoteric surfactant and the fatty alcohol are cooled and prepared in any solid form desired such as block, granule, flake or powdered form. The following exemplifies the present invention:

Example 1

Deriphat® 160C is available from Henkel Corporation and is a nitrogen-containing amphoteric surfactant having the formula as shown for component (a) wherein R contains 12 carbon atoms and having a ratio of sodium atoms to acid hydrogen atoms of 9:1 to 10:1. 30 parts of 30% active Deriphat® 160C (i.e. containing 9 parts of lauriminodipropionate surfactant) is heated to 70°C. Cetyl alcohol at 80 parts is heated to 75°C until clear and

completely melted. The 30% active Deriphat® 160C is then added to the alcohol with continuous agitation provided by a "Lightin" blender until the mixture turns completely clear.

The self-emulsifying cosmetic base is then allowed to cool to a solid form and flaked to a consistency of particles having approximate dimension of 3 mm by 8 mm by 2 mm.

The above example may be repeated using alcohols having a carbon chain length of from 14 to 18.

Potassium, ammonium or a triethyl ammonium derivative may be substituted for sodium in the above example. Similarly, the weight ratio of the salt forming cation to the hydrogen in the nitrogen containing surfactant may be varied between 1:1 and $1:10^{-10}$ with substantially similar results obtained. The products of this Example may also be prepared by spray-drying.

Example II

A hand and body lotion is prepared by combining 3.6 parts of a self-emulsifying cosmetic base (prepared according to the method of Example I from 3.2 parts cetyl alcohol and 0.4 parts Deriphat® 160C) into the following formula:

| | Parts |
|---|---|
| Light Mineral Oil | 5.0 |
| *Generol® 122E5 (Henkel Corporation (PEG—5 Soya Sterol) | 2.0 |
| Stearic acid XXX | 3.0 |
| Cetyl alcohol | 3.2 |
| *Generol® 122E10 (PEG—10 Soya Sterol) | 0.5 |
| *Deriphat® 160C | 0.4 |
| Glycerine | 2.5 |
| Sorbitol | 2.5 |
| Water | to 100 |

*These are products of Henkel Corporation.

The foregoing hand and body lotion is prepared by combining the remaining components at 60°C with the 3.6 parts of the self-emulsifying cosmetic base at 65°C with moderate agitation.

The foregoing provides an ideal hand and body lotion utilising less of the nitrogen containing amphoteric surfactant than was previously known to be useful in forming the emulsion.

Example III

A make-up base is formed by combining 6.75 parts of a self-emulsifying cosmetic base (prepared according to the method of Example I from 6.0 parts cetyl alcohol and 0.75 parts Deriphat® 160C) into the following formula:

| | Parts |
|---|---|
| *Generol® 122E25 (PEG—25 Soya Sterol) | 10.0 |
| *Generol® 122E5 (PEG—5 Soya Sterol) | 2.0 |
| Mineral Oil, light | 4.0 |
| Isopropyl myristate | 2.0 |
| Cetyl alcohol | 6.0 |
| *Deriphat® 160C | 0.75 |
| Propylene glycol | 8.0 |
| Water, deionised | to 100 |

*These are products of Henkel Corporation.

The composition is prepared by heating the self-emulsifying cosmetic base to 75°C and heating the remaining components to 70°C and adding the self-emulsifying cosmetic base thereto.

The make-up base may be utilised alone as described or may have other materials added to it such as pigments including Whittaker #141 Alpine Talc. U.S.P., titanium dioxide and iron oxides. The inclusion of the pigmentation ingredients is best accomplished at temperatures higher than that utilised above; thus the primary make-up base should be heated to 90°C to incorporate the pigments.

## Claims

1. A solid form cosmetic base having self-emulsifying properties and comprising:

a) a mixture of compounds of the formula:

$$MOOCCH_2CH_2NRCH_2CH_2COOM$$

wherein R is a $C_{10}$ to $C_{24}$ fatty alkyl group and M consists of salt forming cations and hydrogen in a respective weight ratio of from 1:1 to $1:10^{-10}$; and

b) a saturated $C_{10}$ to $C_{24}$ fatty alcohol; the weight ratio of component (a) to component (b) being from 1/10:1 to 2/13:1.

2. A cosmetic base according to claim 1 wherein component (a) comprises compounds in which R contains from 12 to 18 carbon atoms.

3. A cosmetic base according to either of claims 1 and 2 wherein the salt forming cation portion of M in component (a) is selected from sodium, potassium, ammonium and alkyl ($C_{1-3}$) derivatives of ammonium either fully or partially replacing the hydrogen atoms on the ammonium and mixtures thereof.

4. A cosmetic base according to any one of the preceding claims wherein component (a) contains the salt forming cations and the hydrogen in a respective weight ratio of from $1:10^{-1}$ to $1:10^{-9}$.

5. A cosmetic base according to any one of the preceding claims wherein component (b) is a fatty alcohol containing from 12 to 18 carbon atoms.

6. A cosmetic base according to any one of the preceding claims wherein component (b) is a monohydric fatty alcohol.

7. A cosmetic base according to any one of the preceding claims wherein the R group of component (a) and the fatty alcohol of

component (b) contain the same number of carbon atoms.

8. A cosmetic base according to any one of the preceding claims wherein the weight ratio of component (a) to component (b) is from 1/9:1 to 1/7:1.

9. A cosmetic composition in the form of an oil-in-water emulsion and made from a cosmetic base as claimed in any of the preceding claims.

**Revendications**

1. Base cosmétique sous forme solide ayant des propriétés auto-émulsifiantes et comprenant:

a) un mélange de composés de formule:

$$MOOCCH_2CH_2NRCH_2CH_2COOM$$

où R est un groupe alkyle gras en $C_{10}$ à $C_{24}$ et M consiste en des cations salifiables et en hydrogène dans un rapport pondéral respectif de 1:1 à $1:10^{-10}$; et

b) un alcool gras saturé en $C_{10}$ à $C_{24}$; le rapport pondéral du composant (a) au composant (b) étant de 1/10:1 à 2/13:1.

2. Base cosmétique selon la revendication 1, où le composant (a) comprend des composés dans lesquels R contient de 12 à 18 atomes de carbone.

3. Base cosmétique selon l'une ou l'autre des revendications 1 et 2, où la partie cation salifiable de M dans le composant (a) et choisie parmi le sodium, le potassium, l'ammonium et des dérivés alkyliques ($C_{1-3}$) de l'ammonium, en remplaçant totalement ou partiellement les atomes d'hydrogène sur l'ammonium, et leurs mélanges.

4. Base cosmétique selon l'une quelconque des revendications précédentes, où le composant (a) contient les cations salifiables et l'hydrogène dans un rapport pondéral respectif de $1:10^{-1}$ à $1:10^{-9}$.

5. Base cosmétique selon l'une quelconque des revendications précédentes, où le composant (b) est un alcool gras contenant de 12 à 18 atomes de carbone.

6. Base cosmétique selon l'une quelconque des revendications précédentes, où le composant (b) est un monoalcool gras.

7. Base cosmétique selon l'une quelconque des revendications précédentes, où le groupe R du composant (a) et l'alcool gras du composant (b) contiennent le même nombre d'atomes de carbone.

8. Base cosmétique selon l'une quelconque des revendications précédentes, où le rapport pondéral du composant (a) au composant (b) est de 1/9:1 à 1/7:1.

9. Composition cosmétique sous la forme d'une émulsion huile dans l'eau et réalisée à partir d'une base cosmétique comme il est revendiqué dans l'une quelconque des revendications précédentes.

**Patentansprüche**

1. Kosmetikgrundstoff in fester Form mit selbstemulgierenden Eigenschaften, enthaltend:

(a) ein Gemisch aus Verbindungen der Formel

$$MOOCCH_2CH_2NRCH_2CH_2COOM$$

worin R eine $C_{10}$- bis $C_{24}$- Fettalkylgruppe bedeutet und M aus salzbildenden Kationen und Wasserstoff besteht, in einem Gewichtsverhältnis von 1:1 bis $1:10^{-10}$ und

(b) einen gesättigten $C_{10}$- bis $C_{24}$-Fettalkohol, wobei das Gewichtsverhältnis von Komponente (a) zu Komponente (b) 1/10:1 bis 2/13:1 beträgt.

2. Kosmetikgrundstoff nach Anspruch 1, worin Komponente (a) Verbindungen enthält, in denen R 12 bis 18 C-Atome enthält.

3. Kosmetikgrundstoff nach einem der Ansprüche 1 oder 2, worin der salzbildende Kationenanteil von M in Komponente (a) Natrium, Kalium, Ammonium, Alkyl-($C_{1-3}$) derivate von Ammonium, die die Wasserstoffatome an Ammonium voll oder teilweise ersetzen, oder Gemische davon ist.

4. Kosmetikgrundstoff nach einem der vorstehenden Ansprüche, worin Komponente (a) die salzbildenden Kationen und den Wasserstoff im Gewichtsverhältnis von $1:10^{-1}$ bis $1:10^{-9}$ enthält.

5. Kosmetikgrundstoff nach einem der vorstehenden Ansprüche, worin Komponente (b) ein Fettalkohol mit 12 bis 18 C-Atomen ist.

6. Kosmetikgrundstoff nach einem der vorstehenden Ansprüche, worin Komponente (b) ein einwertiger Fettalkohol ist.

7. Kosmetikgrundstoff nach einem der vorstehenden Ansprüche, worin die R-Gruppe der Komponente (a) und der Fettalkohol der Komponente (b) die gleiche Anzahl an C-Atomen enthalten.

8. Kosmetikgrundstoff nach einem der vorstehenden Ansprüche, worin das Gewichtsverhältnis von Komponente (a) zu Komponente (b) von 1/9:1 bis 1/7:1 beträgt.

9. Kosmetische Zusammensetzung in Form einer Öl-in-Wasser-Emulsion, hergestellt aus einem Kosmetikgrundstoff gemäß einem der vorstehenden Ansprüche.